# EUROPÄISCHE PATENTSCHRIFT

(11) **EP 2 862 533 B1**
(45) Veröffentlichungstag und Bekanntmachung des Hinweises auf die Patenterteilung: **18.09.2019**
(21) Anmeldenummer: 13189340.6
(22) Anmeldetag: 18.10.2013
(51) Int. Cl.: A61B 18/14, A61B 18/00, A61B 17/3203, A61B 17/32

(54) **Adapterelement, HF-Chirurgieinstrument, Adapteraufsatz und System**
Adapter element, HF surgery instrument, adapter set and system
Élément d'adaptateur, instrument chirurgical HF, jeu d'adaptateurs et système

(43) Veröffentlichungstag der Anmeldung: 22.04.2015
(73) Patentinhaber: Erbe Elektromedizin GmbH, 72072 Tübingen (DE)
(72) Erfinder: Hagg, Martin, 72827 Wannweil (DE)
(74) Vertreter: Bohnenberger, Johannes

(56) Entgegenhaltungen:
- WO-A1-96/23448
- AU-A1- 2008 203 348
- DE-A1-102010 015 899
- DE-A1-102010 060 336
- GB-A- 2 406 793
- US-A- 5 154 709
- US-A- 5 431 650
- US-A- 5 674 219
- US-A- 5 693 044
- US-A- 5 707 402
- US-A1- 2005 119 652

## Beschreibung

Die Erfindung betrifft ein Adapterelement zur Aufnahme eines HF-chirurgischen Handgriffs, der mindestens einen Stromanschluss aufweist, gemäß dem Oberbegriff des Anspruchs 1, ein HF-Chirurgieinstrument mit einem HF-chirurgischen Handgriff und einem Adapterelement zur Aufnahme des Handgriffs gemäß dem Oberbegriff des Anspruchs 13 sowie ein Set gemäß Anspruch 17. Die Erfindung betrifft ferner ein Elektrochirurgiesystem.

HF-chirurgische Einrichtungen der hier angesprochenen Art sind grundsätzlich bekannt, beispielsweise aus US 5,693,044 A, US 5,431,650 A und AU 2008 203 348 A1.

Bei der Anwendung elektrochirurgischer Instrumente, insbesondere beim Koagulieren und Schneiden von Gewebe mit Hilfe von hochfrequentem Strom, erhitzt sich das behandelte Gewebe sehr schnell. Durch die Erhitzung kommt es zu einer Rauchentwicklung, wobei der Rauch feine Partikeln umfassen kann. Der entstehende Rauch besteht nicht nur aus Wasserdampf und Aerosol, sondern kann auch karzinogene und mutagene Inhaltsstoffe aufweisen, die für die im Operationssaal anwesenden Personen gesundheitsschädlich sein können. Während einer elektrochirurgischen Anwendung ist es daher notwendig, Rauchgase unmittelbar am Ursprungsort abzusaugen, um eine zu starke Kontaminierung der Umgebung, insbesondere der Operationssaal und darin
befindliches Personal, zu vermeiden und die damit verbundenen Gefahren für die Gesundheit zu reduzieren. Aus diesem Grund wurden Rauchgas-Absaugsysteme geschaffen, die häufig jedoch unzufriedenstellende Ergebnisse im Hinblick auf die Absaugleistung liefern und darüber hinaus die Ergonomie der elektrochirurgischen Instrumente beeinträchtigen und teilweise sogar die Sicht auf die Operationsstelle behindern.

Aufgabe der vorliegenden Erfindung ist es daher, ein Rauchgas-Absaugsystem in Form eines Adapterelements, eines HF-Chirurgieinstruments und eines Adapteraufsatzes zu schaffen, die eine optimale Absaugleistung mit einer geringstmöglichen Beeinträchtigung von Ergonomie und Sicht bereitstellen.

Zur Lösung der oben genannten Aufgabe wird ein Adapterelement mit den Merkmalen des Anspruchs 1 vorgeschlagen. Das Adapterelement dient zur Aufnahme eines HF-chirurgischen Handgriffs und zeichnet sich dadurch aus, dass ein länglicher Hohlraum zur Aufnahme des Handgriffs und zur Ausbildung des Rauchgaskanals vorgesehen ist, wobei der Hohlraum zur Aufnahme des Handgriffs im Wesentlichen halbschalenförmig ausgebildet ist, so dass nach dem Einsetzen des Handstücks in das Adapterelement der Rauchgaskanal durch einen Außenwandungsbereich des Handgriffs und einen Innenwandungsbereich des Adapterelements gebildet ist.

Der Kern der Erfindung liegt also darin, dass erst durch das Zusammenfügen des Adapterelements mit dem Handgriff der Rauchgaskanal geschlossen wird und durch einen Bereich der Außenwandung des Handgriffs und einen Bereich der Innenwandung des Adapterelements gebildet wird. Auf diese Weise wird nicht nur Material eingespart, sondern die Zusammenfügung des Adapterelements mit dem HF-chirurgischen Handgriff ermöglicht eine gesteigerte Absaugleistung von Rauchgas durch einen größeren Rauchgaskanalquerschnitt sowie eine optimale Ergonomie des resultierenden HF-chirurgischen Instruments. Weiterhin erlaubt das variabel ausgebildete Adapterelement die Verwendung eines Handgriffs für nahezu jeden beliebigen HF-chirurgischen Eingriff und eine daran jeweils angepasste optimale Rauchgasabsaugung. So können für unterschiedliche Einsatzzwecke unterschiedliche Adapterelemente vorgesehen sein, die insbesondere unterschiedliche Elektrodenformen und daran angepasste Rauchgasabsaugungen aufweisen. Für jeden Einsatzzweck kann somit das passende Instrument durch die Kombination des Handgriffs und des Adapterelements realisiert werden. Bei den Adapterelementen kann es sich im Übrigen um Einweg-Produkte handeln, die nach einer einmaligen Benutzung entsorgt werden.

Am distalen Ende des Adapterelements ist erfindungsgemäß mindestens eine stab-, punkt-, ring-, kugel- oder schlingenförmige Elektrode vorgesehen. Es können auch mehrere Elektroden vorgesehen sein, bei denen es sich dann um Kombinationen der vorgenannten verschiedenen Elektrodentypen handeln kann. Um den Anwendungsbereich des Adapterelements noch zu erweitern, kann auch vorgesehen sein, dass er zusätzlich zu der mindestens einen Elektrode einen Flüssigkeitsstrahlapplikator aufweist, der ein Fluid zum Unterspritzen oder Schneiden von Gewebe, z.B. Wasser, bereitstellen kann. Der Flüssigkeitsstrahlapplikator ist vorzugsweise so ausgebildet, dass das distale Ende der Elektrode eine integrierte Düse zum Erzeugen eines Fluidstrahls aufweist. Die Elektrode weist in diesem Fall also eine integrierte Fluidleitung auf und dient somit gleichzeitig als Flüssigkeitszuführung. Alternativ kann auch eine von der Elektrode getrennte Flüssigkeitszufuhrleitung vorgesehen sein. Der HF-chirurgische Handgriff muss dann zusätzlich zu dem Stromanschluss einen Fluidanschluss aufweisen, der dem Flüssigkeitsstrahlapplikator ein Fluid zuführt.

Alternativ oder gegebenenfalls zusätzlich kann das Adapterelement Mittel zur Zuführung von Gas, insbesondere Edelgas, aufweisen. Dadurch ist es möglich mit dem erfindungsgemäßen Chirurgieinstrument auch eine Argonplasmakoagulation durchzuführen.

Vorzugsweise ist vorgesehen, dass der Rauchgaskanal am distalen Ende des Adapterelements in ein Rauchgas-Lumen mündet. Das Rauchgas-Lumen ist zu einem distalen Ende der mindestens einen Elektrode hin geöffnet und dient zur Aufnahme von im Bereich der Elektrode entstehendem Rauchgas und zur Weiterleitung desselben in den Rauchgaskanal. Vorzugsweise ist vorgesehen, dass das Rauchgas-Lumen zur bestmöglichen Absaugung von Rauchgas die Elektrode zumindest bereichsweise umgibt, wobei die Elektrode und das Rauchgas-Lumen konzentrisch oder exzentrisch zueinander angeordnet sein können. Besonders vorteilhaft ist es, wenn das Rauchgas-Lumen an die Form der mindestens einen Elektrode angepasst ist. Beispielsweise kann für eine kugelförmige Elektrode ein halbkugel- oder trichterförmiges oder konisches Rauchgas-Lumen vorgesehen sein, während für eine Stabelektrode beispielweise ein zylindrisches Rauchgas-Lumen vorgesehen sein kann.

Zur Aufnahme des Handgriffs in dem Adapterelement ist ein länglicher Hohlraum vorgesehen, der nicht nur zur Aufnahme des Handgriffs, sondern darüber hinaus zur Ausbildung des Rauchgaskanals dient. Der Hohlraum weist vorzugsweise eine Auflagefläche auf, insbesondere in Form wenigstens einer Kante, einer Schale oder eines Vorsprungs, die zur Lagerung des Handgriffs in bzw. an dem Adapterelement dient. Zum Befestigen des Handgriffs weist das Adapterelement vorzugsweise ein Clip-Element auf, das am proximalen Ende des Adapterelements, zum Einclipsen oder Einrasten des Handgriffs, vorgesehen ist. Auf diese Weise kann der HF-chirurgische Handgriff besonders einfach mittels eines einzigen Bewegungsablaufs in das Adapterelement eingeclipt werden. Besonders vorteilhaft ist es auch, wenn das Adapterelement mit einem Rauchgas-Detektor versehen ist, wobei der Rauchgas-Detektor beispielsweise in dem Rauchgas-Lumen oder in dem Rauchgaskanal, angeordnet sein kann. An seinem proximalen Ende mündet der Rauchgaskanal vorzugsweise in einen Absaugschlauch, der wiederum mit einer geeigneten Absaug- und Filtereinrichtung verbunden ist.

Die mindestens eine Elektrode und das Rauchgas-Lumen können bei einer bevorzugten Ausführungsform des Adapterelements Bestandteil eines Adapteraufsatzes sein, der mit dem Adapterelement austauschbar verbindbar ist. Hierdurch ergibt sich der Vorteil, dass jede Elektrodenform immer mit einem an diese Elektrodenform angepassten Rauchgas-Lumen zusammenwirkt, so dass bei verschiedenen Anwendungen für die unterschiedliche Elektrodenformen von Vorteil sind optimale Absaugleistungen bereitgestellt werden können. Hierbei muss dann für unterschiedliche Elektrodentypen nicht das gesamte Adapterelement ausgetauscht werden, sondern lediglich der Adapteraufsatz ausgetauscht werden.

Zur Lösung der oben genannten Aufgabe wird auch ein HF-Chirurgieinstrument vorgeschlagen, welches einen HF-chirurgischen Handgriff und ein Adapterelement zur Aufnahme des Handgriffs, insbesondere gemäß der Erfindung, aufweist. Das HF-Chirurgieinstrument zeichnet sich dadurch aus, dass ein Innenwandungsbereich des Adapterelements und ein Außenwandungsbereich des Handgriffs im zusammengefügten Zustand des Adapterelements und des Handgriffs einen Rauchgaskanal begrenzen. Hierdurch wird eine besonders hohe Absaugleistung von Rauchgas bewerkstelligt, da der Rauchgaskanal einen größeren Durchmesser aufweisen kann, ohne dass die Ergonomie des Chirurgie-Instruments und die Sicht auf den OP-Situs beeinträchtigt werden.

Besonders vorteilhaft ist es, wenn zwischen dem Adapterelement und dem Handgriff eine vorzugsweise vollständig umlaufende (geschlossene) Dichtung anordenbar ist. In diesem Fall kann ein besonders dichter, insbesondere ein gasdichter Abschluss, zwischen dem Adapterelement und dem Handgriff erfolgen. Der Handgriff weist vorzugsweise mindestens einen Stromanschluss und ggf. auch mindestens einen Fluidanschluss auf. Sofern sowohl ein Stromanschluss als auch ein Fluidanschluss und ein entsprechender Flüssigkeitsstrahlapplikator vorgesehen ist, können mit einem einzigen multifunktionalen HF-Chirurgieinstrument sowohl ein monopolarer Schneidvorgang als auch eine Wasserstrahlapplikation (z.B. Unterspritzen von Gewebe) ohne einen Instrumentenwechsel realisiert werden. Der Handgriff weist vorzugsweise eine Schalteinrichtung zum Steuern der Strom- und/oder Fluidzufuhr auf. Besonders vorteilhaft ist es, wenn zusätzlich eine Verstelleinrichtung zum Verlagern einer aus- und einfahrbaren Elektrode vorgesehen ist.

Zur Lösung der oben genannten Aufgabe wird auch ein Set mit den Merkmalen des Anspruchs 16 vorgeschlagen. Das Set weist die folgenden Bestandteile auf: mindestens einen Adapteraufsatz mit mindestens einer Elektrode und einem Rauchgas-Lumen, mindestens einen Handgriff mit mindestens einem Stromanschluss und ggf. mit mindestens einem Fluidanschluss; und entweder mindestens einen in den Handgriff integrierten oder daran befestigten Rauchgaskanal oder alternativ ein einen Rauchgaskanal aufweisendes Adapterelement zur Aufnahme des Handgriffs. Bei beiden Alternativen wirkt der Rauchgaskanal im zusammengesetzten Zustand des Sets vorzugsweise mit dem Rauchgas-Lumen des Adapteraufsatzes zusammen, um eine optimale Rauchgasabsaugung zu realisieren.

Der Adapteraufsatz ist zur austauschbaren Verbindung mit einem HF-chirurgischen Handgriff mit integrierter Rauchgas-Absaugung und/oder einem Adapterelement mit einem Rauchgaskanal zur Aufnahme eines HF-chirurgischen Handgriffs vorgesehen.

Zum Verbinden des Adapteraufsatzes mit einem Handgriff oder einem Adapterelement ist das proximale Ende des Adapteraufsatzes vorzugsweise an die Form des jeweiligen distalen Endes des Handgriffs oder des Adapterelements angepasst, so dass eine formschlüssige Verbindung zwischen den beiden Teilen entsteht. Weiterhin ist vorzugsweise eine Verbindungseinrichtung, beispielsweise in Form von Rastausnehmungen, einer Schraubverbindung oder einer magnetischen Verbindung vorgesehen, die an dem Adapteraufsatz angeordnet ist und zur Verbindung mit einem HF-chirurgischen Handgriff und/oder einem Adapterelement zur Aufnahme eines HF-chirurgischen Handgriffs dient. Bei den Adapteraufsätzen kann es sich insbesondere um Einweg-Produkte handeln, die nach einmaliger Benutzung entsorgt werden.

Ein Adapteraufsatz kann mindestens eine Elektrode aufweisen, die stab-, punkt-, ring-, kugel- oder schlingenförmig ausgebildet sein kann. Weiterhin können auch Kombinationen von mehreren der vorgenannten Elektrodentypen vorgesehen sein, wobei die mindestens eine Elektrode vorzugsweise verschieblich gelagert ist, so dass also die Elektrodenspitze mehr oder weniger weit aus dem Rauchgas-Lumen herausragen kann.

Der Adapteraufsatz kann darüber hinaus derart ausgebildet sein, dass er eine Doppelfunktion realisiert. So kann zusätzlich zu der Elektrode ein Flüssigkeitsstrahlapplikator vorgesehen sein, der insbesondere derart ausgebildet ist, dass das distale Ende der Elektrode eine integrierte Düse zum Erzeugen eines Fluidstrahls aufweist. In diesem Fall können ohne einen Instrumentenwechsel bzw. einen Wechsel des Adapteraufsatzes mit einem einzigen Adapteraufsatz zwei verschiedene chirurgische Anwendungen realisiert werden. Beispielsweise kann mit der Elektrode koaguliert oder geschnitten und durch die Applikation eines sehr dünnen Wasserstrahls Gewebe unterspritzt werden.

Ferner wird ein Elektrochirurgiesystem mit einem HF-Chirurgieinstrument und einem HF-Generator, der mit dem HF-Chirurgieinstrument verbunden oder verbindbar ist, offenbart und beansprucht. Das HF-Chirurgieinstrument ist erfindungsgemäß ausgebildet. Es kann den in dieser Anmeldung offenbarten Ausführungsformen entsprechend weitergebildet sein.

Die Erfindung wird im Folgenden anhand der Zeichnung näher erläutert.

Es zeigen:
- Fig. 1: eine perspektivische Darstellung eines HF-Chirurgieinstruments gemäß einem ersten Ausführungsbeispiel der Erfindung;
- Fig. 2: eine perspektivische Darstellung eines HF-Chirurgieinstruments gemäß einem zweiten Ausführungsbeispiel der Erfindung;
- Fig. 3: eine perspektivische Darstellung eines HF-Chirurgieinstruments gemäß einem dritten Ausführungsbeispiel der Erfindung;
- Fig. 4: eine perspektivische Darstellung eines HF-Chirurgieinstruments gemäß einem vierten Ausführungsbeispiel der Erfindung;
- Fig. 5: eine perspektivische Darstellung eines HF-Chirurgieinstruments gemäß einem fünften Ausführungsbeispiel der Erfindung;
- Fig. 6a: eine perspektivische Darstellung eines HF-Chirurgieinstruments gemäß einem sechsten Ausführungsbeispiel der Erfindung;
- Fig. 6b: eine Längsschnittdarstellung des HF-Chirurgieinstruments gemäß Fig. 6a;
- Fig. 6c: eine Querschnittdarstellung entlang der Schnittlinie A-A gemäß Fig. 6b;
- Fig. 6d: eine vergrößerte Detailansicht eines Ausschnitts gemäß Fig. 6c;
- Fig. 7a: eine perspektivische Darstellung eines HF-Chirurgieinstruments gemäß einem siebten Ausführungsbeispiel der Erfindung;
- Fig. 7b: eine Längsschnittdarstellung des HF-Chirurgieinstruments gemäß Fig. 7a;
- Fig. 7c: eine Querschnittdarstellung entlang der Schnittlinie A-A gemäß Fig. 7b;
- Fig. 7d: eine vergrößerte Detailansicht eines Ausschnitts gemäß Fig. 7c;
- Fig. 8a: eine perspektivische Darstellung eines HF-Chirurgieinstruments gemäß einem achten Ausführungsbeispiel der Erfindung;
- Fig. 8b: eine Längsschnittdarstellung des HF-Chirurgieinstruments gemäß Fig. 8a;
- Fig. 8c: eine Querschnittdarstellung entlang der Schnittlinie A-A gemäß Fig. 8b;
- Fig. 8d: eine vergrößerte Detailansicht eines Ausschnitts gemäß Fig. 8c;
- Fig. 9: eine Längsschnittdarstellung durch ein HF-Chirurgieinstrument nach einem neunten nicht erfindungsgemäßen Beispiel;
- Fig. 10: eine perspektivische Darstellung eines HF-Chirurgieinstruments gemäß einem zehnten nicht erfindungsgemäßen Beispiel;
- Fig. 11: eine perspektivische Darstellung eines HF-Chirurgieinstruments gemäß einem elften nicht erfindungsgemäßen Beispiel;
- Fig. 12: eine perspektivische Darstellung eines HF-Chirurgieinstruments gemäß einem zwölften nicht erfindungsgemäßen Beispiel;
- Fig. 13: eine perspektivische Darstellung eines HF-Chirurgieinstruments gemäß einem dreizehnten nicht erfindungsgemäßen Beispiel;
- Fig. 14: eine perspektivische Darstellung eines HF-Chirurgieinstruments gemäß einem vierzehnten nicht erfindungsgemäßen Beispiel;
- Fig. 15a: eine Längsschnittdarstellung eines Adapteraufsatzes des erfindungsgemäßen HF-Chirurgieinstruments gemäß einem nicht erfindungsgemäßen Beispiel; und
- Fig. 15b: eine Querschnittdarstellung des Adapteraufsatzes entlang der Schnittlinie B-B gemäß Fig. 15a.

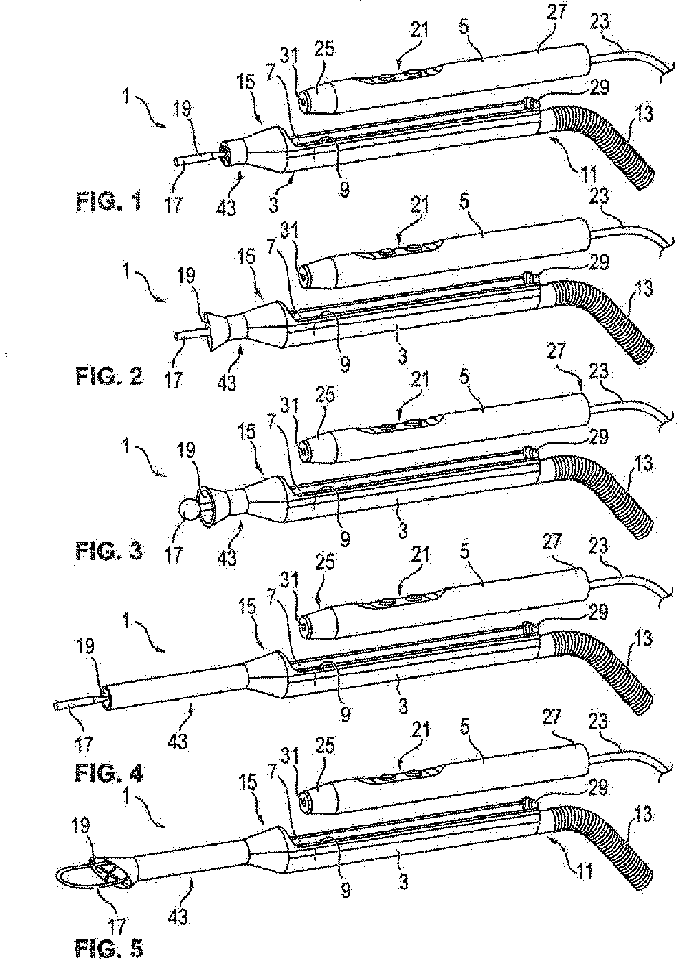

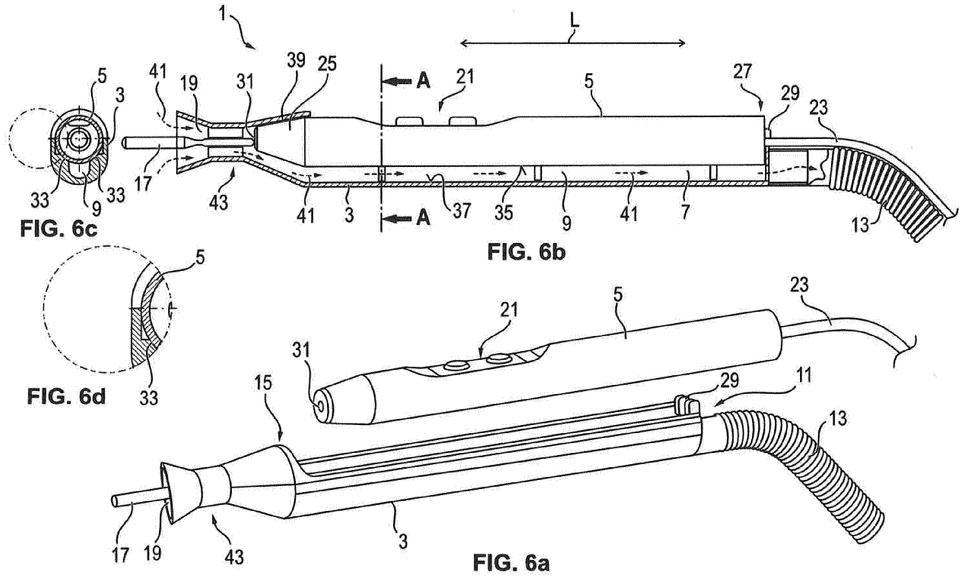

Die Fig. 1 zeigt eine perspektivische Darstellung eines HF-Chirurgieinstruments 1 gemäß der Erfindung. Das HF-Chirurgieinstrument 1 umfasst ein Adapterelement 3 zur Aufnahme eines HF-chirurgischen Handgriffs 5. Das Adapterelement 3 ist im Wesentlichen länglich ausgebildet und insbesondere an die Kontur des Handgriffs 5 so angepasst, dass die Ergonomie des resultierenden HF-Chirurgieinstruments (HF-chirurgisches Instrument) nicht beeinträchtigt wird. Weiterhin weist das Adapterelement 3 einen Hohlraum 7 zur Aufnahme des Handgriffs 5 und zur Ausbildung eines Rauchgaskanals 9 (siehe Fig. 6b) auf.

An einem proximalen Ende 11 des Adapterelements 3 ist ein Absaugschlauch 13 vorgesehen, der einerseits mit dem Hohlraum 7 in Fluidverbindung steht und der andererseits mit einer Absaugeinrichtung, wie einer Pumpe oder dergleichen und einer Filtereinrichtung (in der Figur nicht gezeigt) verbunden ist. An seinem distalen Ende 15 weist das Adapterelement 3 beispielhaft eine stabförmige Elektrode 17 auf sowie ein Rauchgas-Lumen 19 zur Aufnahme von im Bereich der Elektrode entstehendem Rauchgas, wobei die Elektrode 17 das Rauchgas-Lumen 19 durchgreift.

Das Rauchgas-Lumen 19 ist an seinem distalen Ende, d.h. an der dem distalen Elektrodenende zugewandten Seite hin offen und kann auf diese Weise während der Behandlung Rauchgas aufnehmen. Das Rauchgas wird ausgehend von dem Rauchgas-Lumen 19 durch den Rauchgaskanal 9 und den Absaugschlauch 13 gesaugt und in einer geeigneten Filteranlage entsorgt. Vorteilhafterweise ist das Rauchgas-Lumen so ausgebildet, dass es an die jeweilige Elektrodenform angepasst ist. Dieser Sachverhalt wird insbesondere durch die weiteren Figuren 2-5 deutlich, die Adapterelemente mit unterschiedlichen Rauchgas-Lumen 19 und unterschiedlichen Elektroden 17 zeigen, wobei jeweils die Form des Rauchgas-Lumens 19 in optimaler Weise an die Elektrodenform angepasst ist, um im Bereich der Elektrode entstehendes Rauchgas möglichst vollständig aufzunehmen. So ist beispielsweise in der Fig. 2 das Rauchgas-Lumen 19 als länglicher Schlitz ausgebildet, während in der Fig. 3 eine trichterförmige bzw. konische Ausbildung besteht, die zur kugelförmigen Elektrode 17 hin breiter wird. Entsprechendes gilt auch für die Fig. 4, wo ein im Wesentlichen zylindrisches Rauchgas-Lumen 19 eine stabförmige Elektrode 17 zumindest bereichsweise umgibt, und für die Fig. 5, in der ein im Wesentlichen ovales trichterförmiges Rauchgas-Lumen 19 einer schlingenförmigen Elektrode zugeordnet ist.

Der Handgriff 5 umfasst eine Schalteinrichtung 21 zum Steuern der Strom- und/oder Fluidzufuhr zu der Elektrode 17 des Adapterelements. Sofern die Elektrode 17 in einer Längsrichtung verlagerbar ausgebildet ist, kann der Handgriff 5 darüber hinaus eine Verstelleinrichtung zur Verlagerung der Elektrode, manuell oder elektrisch, aufweisen. Weiterhin verfügt der Handgriff 5 über einen Stromanschluss 23, der mit einem HF-Generator verbunden ist, um der Elektrode 17 einen HF-Strom zuzuführen. Sofern das HF-Chirurgieinstrument 1 darüber hinaus einen Flüssigkeitsstrahlapplikator aufweist, kann neben dem Stromanschluss 23 auch ein Fluidanschluss (in der Figur nicht gezeigt) vorgesehen sein.

Zum Verbinden des Handgriffs 5 mit dem Adapterelement 3 muss der Handgriff 5 in den Hohlraum 7 des Adapterelements 3 eingesetzt werden. Hierbei wird zunächst das distale Ende 25 des Handgriffs 5 im distalen Bereich 15 des Adapterelements 3 in den Hohlraum 7 eingebracht, das als Widerlager für das distale Ende 25 des Handgriffs 5 dient. Anschließend wird das proximale Ende 27 des Handgriffs 5 nach unten in den Hohlraum 7 gedrückt, bis der Handgriff 5 mit dem Clip-Element 29 zusammenwirkt und der Handgriff 5 folglich fest in dem Adapterelement 3 verankert ist.

Bei dem Clip-Element 29 kann es sich beispielsweise um ein vorgespanntes flexibles Klemmelement handeln, welches den Stromanschluss 23 an dem Adapterelement 3 fixiert. Durch einen geeigneten Kraftaufwand, der auf das Handstück 5 ausgeübt wird, kann dieses dann wieder aus dem Adapterelement 3 entfernt werden. Grundsätzlich sind auch andere Befestigungsmöglichkeiten zur Fixierung des Handstücks 5 in dem Adapterelement 3 denkbar. Beispielsweise ist an eine magnetische Halterung zu denken. Entscheidend ist, dass sich der Handgriff 5 durch einen einzigen Bewegungsablauf besonders einfach mit dem Adapterelement 3 verbinden lässt.

Zur Verbindung des Stromanschlusses 23 mit der Elektrode 17 weist der Handgriff 5 darüber hinaus einen Anschluss 31 auf, in den das proximale Ende der Elektrode 17 während des Einclipsens des Handgriffs 5 in das Adapterelement 3 eingeführt wird. Auf diese Weise kann eine elektrische Verbindung zwischen einem in der Figur nicht gezeigten HF-Generator und der Elektrode 17 hergestellt werden. Gegebenenfalls ist darüber hinaus ein entsprechender Fluidanschluss vorgesehen, sofern die Elektrode gleichzeitig als Wasserstrahlapplikator ausgebildet ist oder ein von der Elektrode unabhängiger Fluidzufuhrkanal vorgesehen ist.

Insgesamt zeigen die Fig. 1-5, dass ein Handstück 5 mit verschieden ausgebildeten Adapterelementen 3 zu den unterschiedlichsten Anwendungen mit nur einem einzigen Handgriff verbunden werden kann. Besonders vorteilhaft ist dabei, dass ein Rauchgaskanal erst durch das Verbinden des Handstücks 5 mit dem Adapterelement 3 geschaffen wird, der mit einem Rauchgas-Lumen 19 zusammenwirkt, welches jeweils an die zugehörige Elektrodenform angepasst ist. Auf diese Weise wird insgesamt ein großes Rauchgaskanalvolumen und damit eine optimale Rauchgas-Absaugung geschaffen.

Die Fig. 6a zeigt eine perspektivische Darstellung der Ausführungsform nach Fig. 2, während Fig. 6b einen Längsschnitt durch das entsprechende HF-Chirurgieinstrument 1 zeigt, bei dem also das Handstück 5 in das Adapterelement 3 eingeclipt ist.

Die Fig. 6b macht deutlich, dass die längliche Kontur des Adapterelements 3 an die im Wesentlichen zylindrische und ebenfalls längliche Ausbildung des Handstücks 5 angepasst ist. Zur Aufnahme des Handstücks 5 ist der Hohlraum 7 im Wesentlichen halbschalenförmig ausgebildet. Zur Lagerung des Handstücks 5 in dem Hohlraum 7 weist das Adapterelement 3 eine Auflagefläche 33 auf, auf der das Handstück 5 gelagert werden kann. Nach dem Einsetzen des Handstücks 5 in das Adapterelement 3 wird der Rauchgaskanal 9 gebildet durch einen Außenwandungsbereich 35 des Handgriffs 5 und einen Innenwandungsbereich 37 des Adapterelements 3.

Der Handgriff 5 ist in dem Adapterelement 3 sicher durch den Haltebereich 39 am distalen Ende 15 gehalten, der als Widerlager für das distale Ende 25 des Handgriffs 5 dient. Das proximale Ende 27 des Handgriffs 5 ist hingegen mit Hilfe des Clip-Elements 29 an dem Adapterelement 3 befestigt. Der Haltebereich 39 ist dabei vorzugsweise derart an das distale Ende 25 des Handgriffs 5 angepasst, dass ein Formschluss entsteht, wenn der Handgriff 5 in das Adapterelement 3 eingefügt wird. Um ein besonders leichtes Einführen des Handgriffs 5 in den Haltebereich 39 zu gewährleisten, ist das distale Ende 25 des Handgriffs 5 vorzugsweise konisch ausgebildet. Mit anderen Worten weist der Handgriff 5 an seinem distalen Ende einen geringeren Durchmesser auf als in den übrigen Bereichen. Der Haltebereich 39 ist hierzu komplementär ausgebildet und kann auf diese Weise das distale Ende 25 des Handgriffs 5 problemlos aufnehmen.

Die Fig. 6b macht noch deutlich, dass im Operationssitus entstehendes Rauchgas entlang der Pfeile 41 in das Rauchgas-Lumen 19 eingesaugt wird und von dort aus weiter entlang der Pfeile 41 durch den Rauchgaskanal 9 zwischen dem Handgriff 5 und dem Adapterelement 3 und schließlich zu dem Absaugschlauch 13 geleitet wird.

Die Fig. 6b macht schließlich noch deutlich, dass die Elektrode 17 in einem Anschlussstück 43 des Adapterelements, insbesondere durch geeignete Haltestege, im Wesentlichen zentrisch gehalten ist und das Anschlussstück 43 somit durchgreift. Am distalen Ende des Haltestücks 43 ist das vorliegend trichterförmig angeordnete Rauchgas-Lumen 19 zur Aufnahme des Rauchgases vorgesehen. Wie insbesondere auch in Verbindung mit den Fig. 4 und 5 deutlich wird, kann die Länge des Anschlussstücks 43 variieren und folglich für unterschiedliche Anwendungszwecke unterschiedlich ausgebildet sein. Grundsätzlich denkbar ist auch eine teleskopartig variierbare Länge des Anschlussstücks 43.

Die Fig. 6c macht noch deutlich, dass der Auflagebereich 33 zur Lagerung des Handstücks 5 bei dem in Fig. 6a-6d gezeigten Ausführungsbeispiel im Wesentlichen schalenförmig ausgebildet ist, wobei die Auflagefläche nicht zwangsläufig vollflächig entlang der Längsrichtung L des HF-chirurgischen Instruments ausgebildet sein muss. Denkbar ist nämlich grundsätzlich auch die Anordnung von mehreren in einem Abstand zueinander angeordneten Stegen, die den Rauchgasstrom entlang der Pfeile 41 in dem Rauchgaskanal 9 so wenig wie möglich behindern.

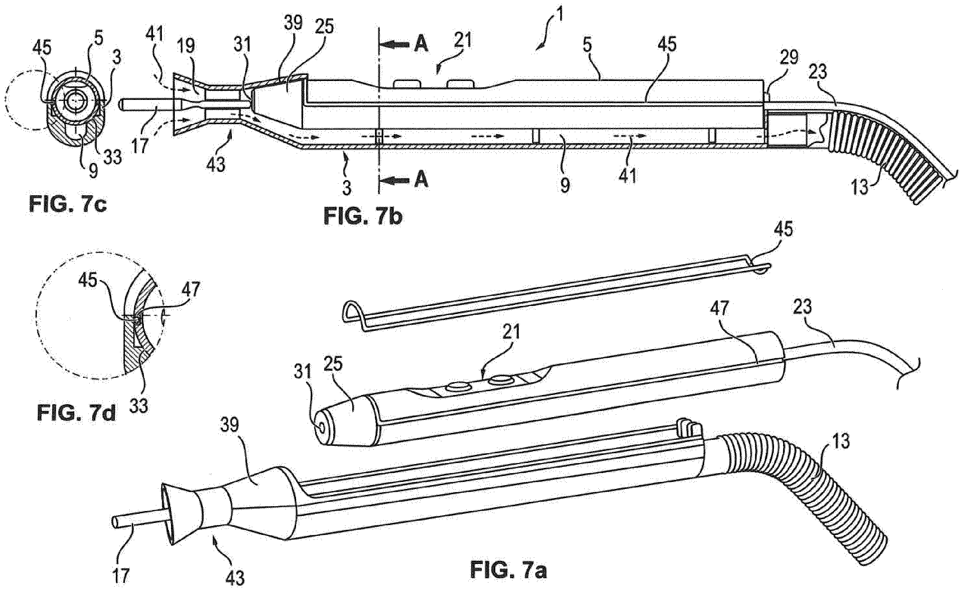

Die Fig. 7a-7d zeigen ein weiteres Ausführungsbeispiel der Erfindung, bei dem zwischen dem Handgriff 5 und dem Adapterelement 3 eine Dichtung 45 vorgesehen ist, die umlaufend geschlossen ausgebildet ist und einen (gasdichten) Abschluss der Verbindung zwischen dem Handgriff 5 und dem Adapterelement 3 gewährleistet. Zur Aufnahme der Dichtung 45 kann der Handgriff 5 eine entsprechend der Form der Dichtung ausgebildete Aufnahmenut 47 (wie in Fig. 7a gezeigt) aufweisen. Denkbar ist es jedoch grundsätzlich auch, eine entsprechende Aufnahmenut im Bereich der Innenwandung des Adapterelements 3 vorzusehen. Wie insbesondere die Fig. 7d deutlich macht, liegt im zusammengefügten Zustand des Handgriffs 5 und des Adapterelements 3 die Dichtung 45 zwischen der Innenwandung 37 des Adapterelements 3 und der Außenwandung 35 des Handgriffs 5 und sorgt damit für eine Abdichtung des Rauchgaskanals nach außen hin.

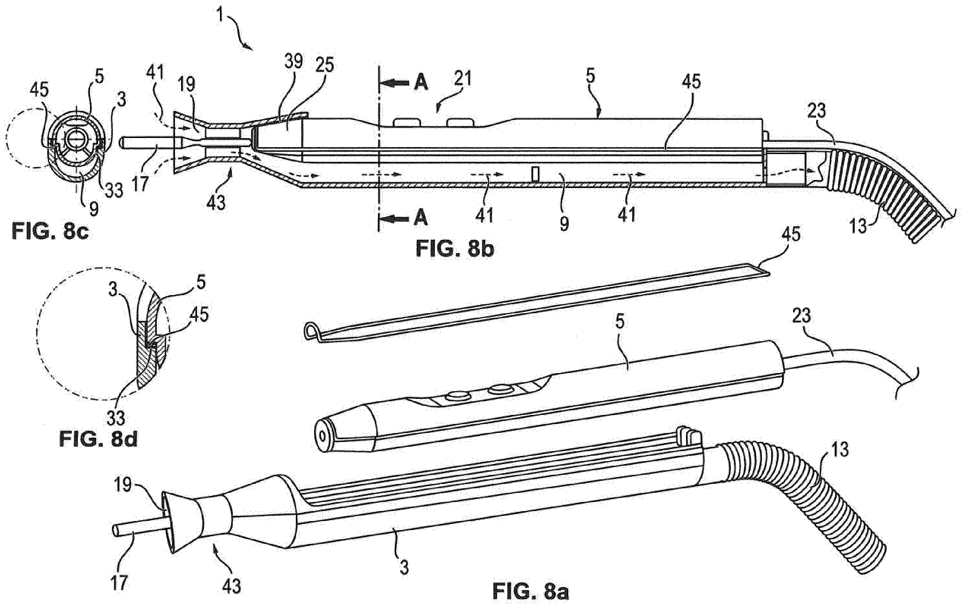

Die Fig. 8a-8d zeigen noch eine weitere Ausführungsform eines HF-Chirurgieinstruments 1, bei dem die Innenwandung des Adapterelements 3 eine in der Längsrichtung L verlaufende Stufe aufweist, auf der eine komplementär ausgebildete Stufe des Handgriffs 5 gelagert ist. Die Lagerung erfolgt bei dem Ausführungsbeispiel nach den Fig. 8a-8d also in Form von Auflageflächen 33, die als Stufen ausgebildet sind und somit die Ausbildung eines deutlich vergrößerten Rauchgaskanalquerschnitts ermöglichen. Auch bei diesem Ausführungsbeispiel der Erfindung ist eine Dichtung 45 vorgesehen, die vorzugsweise zwischen dem Handgriff 5 und dem Adapterelement 3 im Bereich der stufenförmigen Auflagefläche 33 anordenbar ist.

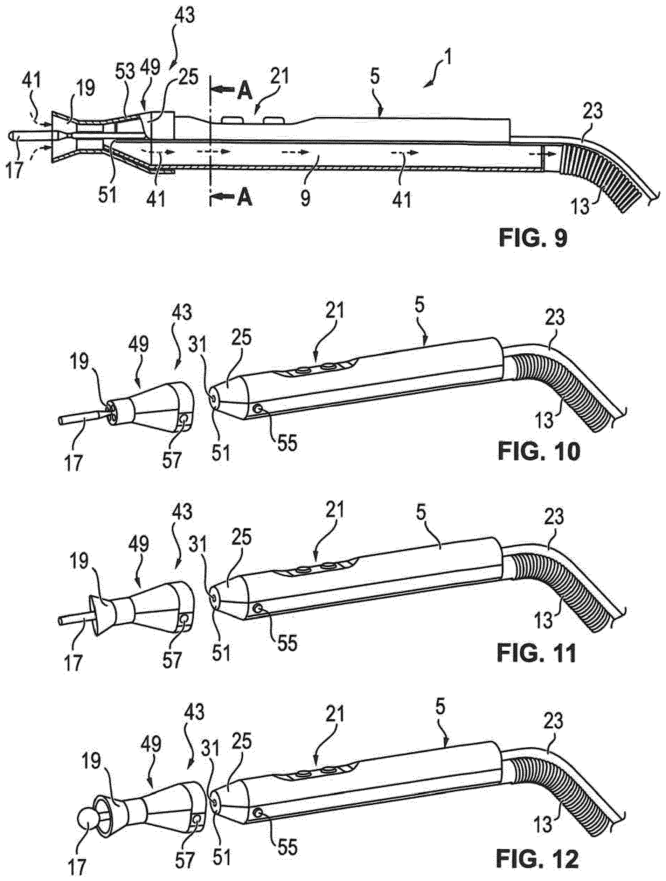

Die Fig. 9-14 zeigen noch unterschiedliche Beispiele eines Adapterelements, bei welcher das Anschlussstück 43 nicht einstückig mit dem Adapterelement 3 ausgebildet ist, sondern in Form eines Adapteraufsatzes 49, der austauschbar mit dem Adapterelement 3 und/oder mit einem herkömmlichen Handgriff 5, insbesondere mit einem Handgriff mit integriertem oder daran befestigbarem Rauchgaskanal, verbindbar ist.

Die Fig. 9 zeigt einen Längsschnitt durch ein HF-Chirurgieinstrument 1 mit einem Handgriff 5 und einem darin integrierten Rauchgaskanal 9, der beispielsweise auch als Schlauch ausgebildet sein kann, der am distalen Ende 25 des Handgriffs 5 eine Öffnung 51 aufweist, die eine Fluidverbindung mit dem Adapteraufsatz 49 ermöglicht. Der Adapteraufsatz 49 umfasst, wie das Anschlussstück 43 der Fig. 1-8, eine Elektrode 17, die, beispielsweise mit Hilfe von Stegen oder dergleichen Befestigungsmitteln, zentrisch oder exzentrisch im Inneren des Adapteraufsatzes 49 gehalten ist und aus einem Rauchgas-Lumen 19 herausragt.

Der Adapteraufsatz 49 wird auf den Handgriff bzw. das Handstück 5 aufgeschoben, so dass das proximale Ende 53 in den elektrischen Anschluss 31 des Handgriffs 5 eingreift, um eine elektrische Verbindung zwischen einem HF-Generator und der Elektrode 17 herzustellen. Wie die Fig. 9 zeigt, ist das proximale Ende des Adapteraufsatzes 49 an die Form des distalen Endes 25 des Handgriffs 5 so angepasst, dass bei einer Verbindung der beiden Teile ein Formschluss entsteht. Auch in diesem Fall kann eine Dichtung vorgesehen sein, die vorzugsweise vollständig umlaufend im Verbindungsbereich zwischen dem Handgriff 5 und dem Adapteraufsatz 49 angeordnet ist.

Eine Fixierung des Adapteraufsatzes 49 an dem Handgriff 5 kann durch geeignete Befestigungsmittel erfolgen. In den Fig. 9-14 ist beispielhaft eine Variante dargestellt, bei der der Adapteraufsatz 49 einfach auf den Handgriff 5 aufgeclipt werden kann, indem an dem Handgriff 5 vorgesehene Rastelemente 55 in entsprechende Rastausnehmungen 57 des Adapteraufsatzes 49 eingeführt werden. Sofern die Rastelemente 55 federgelagert oder flexibel sind, kann ein einfaches Lösen des Adapteraufsatzes 49 von dem Handgriff 5 erfolgen, beispielsweise dann, wenn für einen anderen chirurgischen Eingriff eine Elektrode mit einer anderen Form benötigt wird. Alternativ kann auch vorgesehen sein, dass magnetische oder dergleichen Befestigungsmittel an dem Adapteraufsatz 49 und an dem Handgriff 5 zur Verbindung der beiden Teile vorgesehen sind.

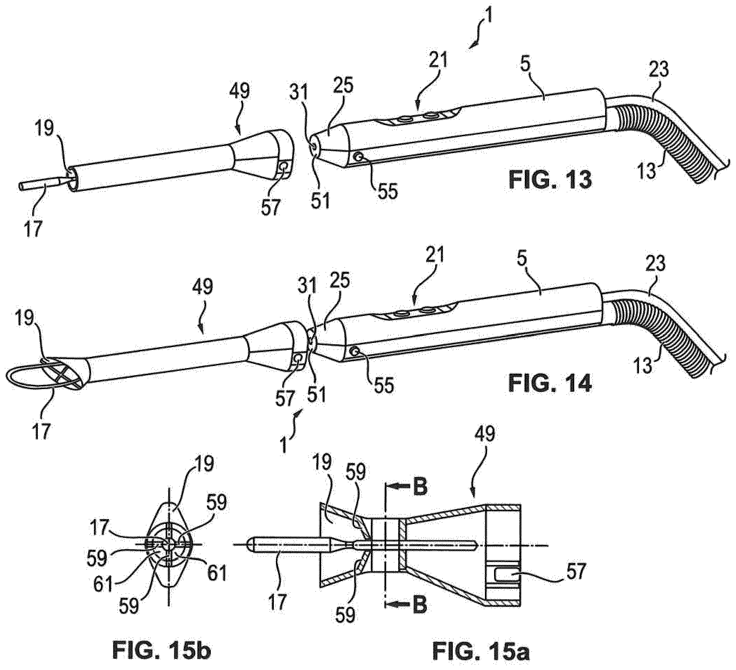

Die Fig. 13 und 14 zeigen weitere Ausführungsbeispiele eines Adapteraufsatzes 49, die deutlich länger ausgebildet sind als die Beispiele gemäß den Fig. 9-12. Insgesamt zeigt sich somit, dass der Adapteraufsatz 49 für die verschiedensten Anwendungsfälle mit unterschiedlichen Längen, unterschiedlichen Elektroden und unterschiedlichen Rauchgas-Lumen ausgebildet sein kann. Ein Austausch ist besonders einfach möglich, indem der Aufsatz einfach auf einen Handgriff 5 aufgeclipst wird. Der Adapteraufsatz 49 kann grundsätzlich auch einen Flüssigkeitsstrahlapplikator aufweisen. Dieser kann beispielsweise in Form einer in die Elektrode integrierte Düse ausgebildet sein oder aber getrennt von der Elektrode in dem Adapteraufsatz 49 ausgebildet sein. Für den Fall, dass der Adapteraufsatz 49 zur Durchführung einer Fluidapplikation (üblicherweise Wasserapplikation) verwendet werden soll, versteht sich, dass der Handgriff 5 eine entsprechende Flüssigkeitszuleitung und entsprechende Steuermittel aufweisen kann. Auch die in den Fig. 9-14 gezeigte Ausführungsform der Erfindung kann im Übrigen eine in der Länge variable Elektrode 17 aufweisen, die also mehr oder weniger weit aus dem Adapteraufsatz 49 herausragen kann. Entsprechende Steuerungsmittel sind in diesem Fall vorzugsweise an dem Handgriff 5 vorgesehen, so dass sich eine Verlagerung der Elektrode 17 manuell oder auch elektrisch vornehmen lässt.

Der Adapteraufsatz 49 ist nicht nur für die in den Fig. 9-14 gezeigte einteilige Ausführungsform des Handgriffs 5 mit einem integrierten Rauchgaskanal 9 vorgesehen, sondern grundsätzlich ist es auch denkbar, den Adapteraufsatz 49 mit einem Adapterelement 3 gemäß den Fig. 1-8 zu verbinden, so dass einerseits das Adapterelement 3 für unterschiedliche Handgriffe 5 ausgebildet sein kann, während der Adapteraufsatz 49 für jedes Adapterelement 3 passend ausgebildet ist. Während also zwischen dem Adapteraufsatz 49 und dem Adapterelement 3 eine Standardschnittstelle bestehen kann, können unterschiedliche Adapterelemente 3 für unterschiedliche Handgriffe 5, insbesondere an unterschiedliche Formen und Abmessungen, angepasst sein. Ein entsprechendes HF-Chirurgieset kann dann beispielsweise ein oder mehrere Adapteraufsätze, ein oder mehrere Adapterelemente (Adapterschale) und einen oder mehrere Handgriffe 5 aufweisen.

Die Fig. 15a zeigt einen Längsschnitt durch einen Adapteraufsatz 49, während Fig. 15b eine Schnittdarstellung entlang der Schnittlinie B-B der Fig. 15a zeigt. Die Fig. 15a und 15b machen deutlich, dass eine Halterung der Elektrode 17 im Inneren des Adapteraufsatzes 49 mit Hilfe von Stegen 59 erfolgen kann, die einerseits mit der Innenwandung des Adapteraufsatzes 49 in Verbindung stehen und die andererseits eine ringförmige Öffnung zur Aufnahme der Elektrode 17 bilden. Zwischen den einzelnen Stegen sind Öffnungen 61 ausgebildet, die ein Durchtreten eines in das Rauchgas-Lumen 19 eingesaugten Rauchgases durch die Öffnung 51 des Handgriffs 5 und von dort aus in den Rauchgaskanal 9 ermöglichen.

Insgesamt schafft die vorliegende Erfindung ein vorteilhaftes Rauch-Absaugsystem, das durch seine modulare Ausbildung nicht nur flexibel an viele unterschiedliche HF-chirurgische Anwendungen anpassbar ist (insbesondere auch kombinierte Anwendungen mit Strom und Flüssigkeit), sondern welches darüber hinaus eine optimale Absaugleistung mit geringstmöglicher Behinderung von Ergonomie und Sicht des Operationsgebietes für jede individuelle elektrochirurgische Anwendung ermöglicht.

### Bezugszeichenliste

- 1: HF-Chirurgieinstrument
- 3: Adapterelement
- 5: Handgriff
- 7: Hohlraum
- 9: Rauchgaskanal
- 11: Proximales Ende
- 13: Absaugschlauch
- 15: Distales Ende
- 17: Elektrode
- 19: Rauchgas-Lumen
- 21: Schalteinrichtung
- 23: Stromanschluss
- 25: Distales Ende
- 27: Proximales Ende
- 29: Clip-Element
- 31: Anschluss
- 33: Auflagefläche
- 35: Außenwandungsbereich
- 37: Innenwandungsbereich
- 39: Haltebereich
- 41: Pfeil
- 43: Anschlussstück
- 45: Dichtung
- 47: Aufnahmenut
- 49: Adapteraufsatz
- 51: Öffnung
- 53: Proximales Ende
- 55: Rastelement
- 57: Rastausnehmung
- 59: Steg
- 61: Öffnung
- L: Längsachse

## Patentansprüche

1. Adapterelement (3) zur Aufnahme eines HF-chirurgischen Handgriffs (5), wobei durch die Aufnahme des Handgriffs (5) in das Adapterelement (3) ein Rauchgaskanal (9) zum Ableiten von Rauchgas aus einem Behandlungsgebiet in dem Adapterelement (3) ausbildbar ist und an seinem distalen Ende mindestens eine stab-, punkt-, ring-, kugel- oder schlingenförmige Elektrode (17) oder Kombinationen derselben angebracht ist/sind
**dadurch gekennzeichnet, dass**
ein länglicher Hohlraum (7) zur Aufnahme des Handgriffs (5) und zur Ausbildung des Rauchgaskanals (9) vorgesehen ist, wobei der Hohlraum (7) zur Aufnahme des Handgriffs (5) im Wesentlichen halbschalenförmig ausgebildet ist, so dass nach dem Einsetzen des Handstücks (5) in das Adapterelement (3) der Rauchgaskanal (9) durch einen Außenwandungsbereich (35) des Handgriffs (5) und einen Innenwandungsbereich (37) des Adapterelements (3) gebildet ist.

2. Adapterelement nach Anspruch 1,
**dadurch gekennzeichnet, dass**
die Elektrode (17) oder die Elektroden (17) verschieblich gelagert ist/sind.

3. Adapterelement nach Anspruch 1 oder 2,
**dadurch gekennzeichnet, dass**
ein Flüssigkeitsstrahlapplikator vorgesehen ist, der insbesondere derart ausgebildet ist, dass das distale Ende der Elektrode (17) eine integrierte Düse zum Erzeugen eines Fluidstrahls aufweist.

4. Adapterelement nach einem der vorhergehenden Ansprüche,
**dadurch gekennzeichnet, dass**
der Rauchgaskanal (9) am distalen Ende (15) des Adapterelements (3) in ein Rauchgas-Lumen (19) mündet.

5. Adapterelement nach Anspruch 4,
**dadurch gekennzeichnet, dass**
das Rauchgas-Lumen (19) zu einem distalen Ende der mindestens einen Elektrode (17) hin geöffnet ist und diese zumindest bereichsweise entweder konzentrisch oder exzentrisch umgibt.

6. Adapterelement nach einem der vorhergehenden Ansprüche 4 oder 5,
**dadurch gekennzeichnet, dass**
das Rauchgas-Lumen (19) an die Form der mindestens einen Elektrode (17) angepasst ist, und insbesondere im Wesentlichen zylindrisch oder konisch ausgebildet ist.

7. Adapterelement nach einem der vorhergehenden Ansprüche,
**dadurch gekennzeichnet, dass**
der Hohlraum (7) eine Auflagefläche (33), insbesondere in Form wenigstens einer Kante, wenigstens eines Stegs oder wenigstens einer Schale, zur Lagerung des Handgriffs (5) aufweist.

8. Adapterelement nach einem der vorhergehenden Ansprüche,
**dadurch gekennzeichnet, dass**
ein Clip-Element (29), vorzugsweise am proximalen Ende des Adapterelements (3), zum Einclipsen oder Einrasten des Handgriffs (5) an dem Adapterelement (3) vorgesehen ist.

9. Adapterelement nach einem der vorhergehenden Ansprüche,
**dadurch gekennzeichnet, dass**
ein Rauchgasdetektor, insbesondere in dem Rauchgas-Lumen (19) oder in dem Rauchgaskanal (9), angeordnet ist.

10. Adapterelement nach einem der vorhergehenden Ansprüche,
**dadurch gekennzeichnet, dass**
der Rauchgaskanal (9) in einen Absaugschlauch (13) mündet, der insbesondere mit dem proximalen Ende (11) des Adapterelements (3) verbunden ist.

11. Adapterelement nach einem der vorhergehenden Ansprüche,
**dadurch gekennzeichnet, dass**
die mindestens eine Elektrode (17) und das Rauchgas-Lumen (19) Bestandteile eines Adapteraufsatzes (49) sind, der mit dem Adapterelement (3) austauschbar verbindbar ist.

12. HF-Chirurgieinstrument, aufweisend einen HF-chirurgischen Handgriff (5) und ein Adapterelement (3) zur Aufnahme des Handgriffs nach einem der vorhergehenden Ansprüche 1 bis 11,
**dadurch gekennzeichnet, dass**
ein Innenwandungsbereich (37) des Adapterelements (3) und ein Außenwandungsbereich (35) des Handgriffs (5) im zusammengefügten Zustand des Adapterelements (3) und des Handgriffs (5) einen Rauchgaskanal (9) begrenzen.

13. HF-Chirurgieinstrument nach Anspruch 12,
**dadurch gekennzeichnet, dass**
zwischen dem Adapterelement (3) und dem Handgriff (5) eine vorzugsweise umlaufende Dichtung (45) anordenbar ist.

14. HF-Chirurgieinstrument nach Anspruch 12 oder 13,
**dadurch gekennzeichnet, dass**
der Handgriff (5) mindestens einen Stromanschluss und/oder mindestens einen Fluidanschluss aufweist.

15. HF-Chirurgieinstrument nach einem der vorhergehenden Ansprüche 12 bis 14,
**dadurch gekennzeichnet, dass**
der Handgriff (5) eine Schalteinrichtung (21) zum Steuern der Strom- und/oder Fluidzufuhr, und insbesondere zusätzlich eine Verstelleinrichtung zum Verlagern der Elektrode, aufweist.

16. Set aufweisend:
- mindestens einen Handgriff (5) mit mindestens einem Stromanschluss (23) und ggf. mit mindestens einem Fluidanschluss; und
- mindestens einen Adapteraufsatz (49) zur austauschbaren Verbindung mit einem HF-chirurgischen Handgriff (5) mit mindestens einer Elektrode (17) und einem Rauchgas-Lumen (19) sowie einem Adapterelement (3) nach einem der Ansprüche 1 bis 11 zur Aufnahme des Handgriffs (5).

17. Elektrochirurgiesystem mit einem HF-Chirurgieinstrument nach Anspruch 12 und einem HF-Generator, der mit dem HF-Chirurgieinstrument verbunden oder verbindbar ist.

## Claims

1. Adapter element (3) for receiving an HF surgical handpiece (5), wherein, by placing the handpiece (5) into the adapter element (3), a smoke duct (9) for carrying off smoke from a treatment region can be formed in the adapter element (3), and at least one rod-shaped, point-shaped, ring-shaped, ball-shaped or loop-shaped electrode (17) or combinations of these is/are mounted at its distal end, **characterized in that** an elongate cavity (7) for receiving the handpiece (5) and for forming the smoke duct (9) is provided, wherein the cavity (7) for receiving the handpiece (5) is substantially in the shape of a half shell such that, after the insertion of the handpiece (5) into the adapter element (3), the smoke duct (9) is formed by an outer wall region (35) of the handpiece (5) and an inner wall region (37) of the adapter element (3).

2. Adapter element according to Claim 1, **characterized in that** the electrode (17) or the electrodes (17) is/are mounted displaceably.

3. Adapter element according to Claim 1 or 2, **characterized in that** a liquid jet applicator is provided, which in particular is configured in such a way that the distal end of the electrode (17) has an integrated nozzle for generating a fluid jet.

4. Adapter element according to one of the preceding claims, **characterized in that** the smoke duct (9), at the distal end (15) of the adapter element (3), opens into a smoke lumen (19).

5. Adapter element according to Claim 4, **characterized in that** the smoke lumen (19) is open towards a distal end of the at least one electrode (17) and at least in part encircles the latter either concentrically or eccentrically.

6. Adapter element according to either of preceding Claims 4 and 5, **characterized in that** the smoke lumen (19) is adapted to the shape of the at least one electrode (17) and in particular has a substantially cylindrical or conical shape.

7. Adapter element according to one of the preceding claims, **characterized in that** the cavity (7) has a bearing surface (33), in particular in the form of at least one edge, at least one web or at least one shell, for supporting the handpiece (5).

8. Adapter element according to one of the preceding claims, **characterized in that** a clip element (29), preferably at the proximal end of the adapter element (3), is provided for clipping or latching the handpiece (5) on the adapter element (3).

9. Adapter element according to one of the preceding claims, **characterized in that** a smoke detector is arranged in particular in the smoke lumen (19) or in the smoke duct (9).

10. Adapter element according to one of the preceding claims, **characterized in that** the smoke duct (9) opens into a suction hose (13), which is connected in particular to the proximal end (11) of the adapter element (3).

11. Adapter element according to one of the preceding claims, **characterized in that** the at least one electrode (17) and the smoke lumen (19) are components of an adapter attachment (49), which is exchangeably connectable to the adapter element (3).

12. HF surgical instrument, having an HF surgical handpiece (5) and an adapter element (3) for receiving the handpiece according to one of preceding Claims 1 to 11, **characterized in that** an inner wall region (37) of the adapter element (3) and an outer wall region (35) of the handpiece (5) delimit a smoke duct (9) in the assembled state of the adapter element (3) and the handpiece (5).

13. HF surgical instrument according to Claim 12, **characterized in that** a preferably peripheral seal (45) can be arranged between the adapter element (3) and the handpiece (5).

14. HF surgical instrument according to Claim 12 or 13, **characterized in that** the handpiece (5) has at least one power connection and/or at least one fluid connection.

15. HF surgical instrument according to one of preceding Claims 12 to 14, **characterized in that** the handpiece (5) has a switching device (21) for controlling the power and/or fluid supply, and in particular additionally an adjusting device for moving the electrode.

16. Set having:
- at least one handpiece (5) with at least one power connection (23) and, if necessary, with at least one fluid connection; and
- at least one adapter attachment (49) for exchangeable connection to an HF surgical handpiece (5) with at least one electrode (17) and a smoke lumen (19), and also an adapter element (3) according to one of Claims 1 to 11 for receiving the handpiece (5).

17. Electrosurgery system with an HF surgical instrument according to Claim 12, and with an HF generator which is connected or connectable to the HF surgical instrument.

## Revendications

1. Elément adaptateur (3) destiné à recevoir une poignée chirurgicale HF (5), un conduit de gaz de combustion (9) destiné à évacuer des gaz de combustion d'une région de traitement pouvant être formé dans l'élément adaptateur (3) lorsque la poignée (5) est reçue dans l'élément adaptateur (3) et au moins une électrode (17) en forme de tige, de point, d'anneau, de sphère ou de boucle ou une combinaison de ceux-ci étant montée à son extrémité distale,
**caractérisé en ce que**
une cavité allongée (7) est prévue pour recevoir la poignée (5) et pour former le conduit de gaz de combustion (9), la cavité (7) destinée à recevoir la poignée (5) étant sensiblement en forme de demi-coque de sorte qu'après l'insertion de la poignée (5) dans l'élément adaptateur (3) le conduit de gaz de combustion (9) est formé par une région de paroi extérieure (35) de la poignée (5) et une région de paroi intérieure (37) de l'élément adaptateur (3).

2. Elément adaptateur selon la revendication 1,
**caractérisé en ce que**
l'électrode (17) ou les électrodes (17) sont montées de manière coulissante.

3. Elément adaptateur selon la revendication 1 ou 2,
**caractérisé en ce que**
un applicateur à jet de liquide est prévu qui est conçu en particulier de telle sorte que l'extrémité distale de l'électrode (17) comporte une buse intégrée destinée à générer un jet de fluide.

4. Elément adaptateur selon l'une des revendications précédentes,
**caractérisé en ce que**
le conduit de gaz de combustion (9) débouche dans une lumière de gaz de combustion (19) à l'extrémité distale (15) de l'élément adaptateur (3).

5. Elément d'adaptation selon la revendication 4,
**caractérisé en ce que**
la lumière de gaz de combustion (19) est ouverte vers une extrémité distale de l'au moins une électrode (17) et entoure celle-ci au moins par endroits de manière concentrique ou excentrique.

6. Elément adaptateur selon l'une des revendications précédentes 4 et 5,
**caractérisé en ce que**
la lumière de gaz de combustion (19) est adaptée à la forme de l'au moins une électrode (17), et a en particulier une forme sensiblement cylindrique ou conique.

7. Elément adaptateur selon l'une des revendications précédentes,
**caractérisé en ce que**
la cavité (7) comporte une surface d'appui (33), se présentant notamment sous la forme d'au moins un bord, d'au moins une nervure ou d'au moins une coque, pour le montage de la poignée (5).

8. Elément adaptateur selon l'une des revendications précédentes,
**caractérisé en ce que**
un élément d'encliquetage (29) est prévu, de préférence à l'extrémité proximale de l'élément adaptateur (3), pour encliqueter ou verrouiller la poignée (5) sur l'élément adaptateur (3).

9. Elément d'adaptation selon l'une des revendications précédentes,
**caractérisé en ce que**
un détecteur de gaz de combustion est disposé en particulier dans la lumière de gaz de combustion (19) ou dans le conduit de gaz de combustion (9).

10. Elément adaptateur selon l'une des revendications précédentes,
**caractérisé en ce que**
le conduit de gaz de combustion (9) débouche dans un tuyau d'aspiration (13) qui est notamment relié à l'extrémité proximale (11) de l'élément adaptateur (3).

11. Elément adaptateur selon l'une des revendications précédentes,
**caractérisé en ce que**
l'au moins une électrode (17) et la lumière de gaz de combustion (19) sont des composants d'un embout adaptateur (49) pouvant être relié de manière interchangeable à l'élément adaptateur (3).

12. Instrument chirurgical HF, comprenant une poignée chirurgicale HF (5) et un élément adaptateur (3) destiné à recevoir la poignée selon l'une des revendications précédentes 1 à 11,
**caractérisé en ce qu'**une région de paroi intérieure (37) de l'élément adaptateur (3) et une région de paroi extérieure (35) de la poignée (5) définissent un conduit de gaz de combustion (9) lorsque l'élément adaptateur (3) et la poignée (5) sont assemblés.

13. Instrument chirurgical HF selon la revendication 12,
**caractérisé en ce que**
une garniture d'étanchéité (45) de préférence circonférentielle peut être disposée entre l'élément adaptateur (3) et la poignée (5).

14. Instrument chirurgical HF selon la revendication 12 ou 13,
**caractérisé en ce que**
la poignée (5) comporte au moins un raccord de courant et/ou au moins un raccord de fluide.

15. Instrument chirurgical HF selon l'une des revendications précédentes 12 à 14,
**caractérisé en ce que**
la poignée (5) comporte un moyen de commutation (21) destiné à commander l'alimentation en courant et/ou en fluide, et en particulier en plus un moyen de réglage destiné à déplacer l'électrode.

16. Ensemble comprenant :
- au moins une poignée (5) pourvue d'au moins un raccord de courant (23) et éventuellement au moins un raccord de fluide ; et
- au moins un embout adaptateur (49) destiné être relié de manière interchangeable à une poignée chirurgicale HF (5) pourvue d'au moins une électrode (17) et une lumière de gaz de combustion (19) ainsi que d'un élément adaptateur (3) selon l'une des revendications 1 à 11 destiné à recevoir la poignée (5) .

17. Système électro-chirurgical comprenant un instrument chirurgical HF selon la revendication 12 et un générateur HF relié ou pouvant être relié à l'instrument chirurgical HF.
